# EUROPEAN PATENT APPLICATION

(11) **EP 2 436 396 A1**
(43) Date of publication of application: **04.04.2012**
(21) Application number: 10780643.2
(22) Date of filing: 28.05.2010
(51) Int. Cl.: A61K 45/00, A61K 31/53, A61P 25/00, A61P 25/14, A61P 25/18, C07D 403/12

(54) **NOVEL PHARMACEUTICAL COMPOSITION FOR PREVENTION AND/OR TREATMENT OF ATTENTION DEFICIT/HYPERACTIVITY DISORDER**

(30) Priority: 29.05.2009 JP 2009129780
(71) Applicant: Astellas Pharma Inc., Tokyo 103-8411 (JP)
(72) Inventor: TAKAHASHI, Shinji, North Deerfield, IL 60015 (US)
(74) Representative: HOFFMANN EITLE
(86) International application number: PCT/JP2010/059085
(87) International publication number: WO 2010/137689

(57) **Abstract**

The present invention is to provide a therapeutic agent for attention deficit/hyperactivity disorder having a novel mechanism of action which is different from conventional psychostimulants. The present invention is useful for providing an excellent pharmaceutical composition for prevention and/or treatment of attention deficit/hyperactivity disorder, comprising a BEC1 potassium channel inhibitor or a pharmaceutically acceptable salt thereof as an active ingredient, and is particularly useful for providing a pharmaceutical composition for prevention and/or treatment of inattention, impulsivity, and hyperactivity in attention deficit/hyperactivity disorder.

## Description

### Technical Field

The present invention relates to a novel pharmaceutical use of a BEG 1 potassium channel inhibitor as a pharmaceutical composition for prevention and/or treatment of attention deficit/hyperactivity disorder.

### Background Art

Attention deficit/hyperactivity disorder is a developmental disorder, of which the three main symptoms are inattention (having difficulty focusing attention on matters and often losing things), hyperactivity (being lack of composure and having difficulty staying still), and impulsivity (putting an impetuous idea into action uninhibitedly and having difficulty waiting one's turn) observed starting before seven years of age (American Psychiatric Association: Diagnostic and Statistical Manual of Mental Disorders, 4th ed., Text Revision: DSM-IV-TR. American Psychiatric Association, Washington, D. C., 2000). Among these main symptoms, hyperactivity and impulsivity are often to be relieved around 11 years old and 13 years old, respectively, however, the symptom of inattention may often persist over a longer period into adulthood. According to recent investigations, the persistence of symptoms are shown to exist in about 70% during adolescence (Barkley, R. A., Fischer, M., Edelbrock, C. S. and Smallish, L., J. Am. Acad. Child Adolesc. Psychiatry, 29: 546-557, 1990), and also in about 50% patients during adulthood (Barkley, R. A., Fischer, M., Smallish, L. and Fletcher, K., J. Abnorm. Psychol. 111: 279-89, 2002). There is a considerable difference in ways of revealing symptoms between individuals, and those can be classified into three types: combined type (three symptoms of inattention, hyperactivity, and impulsivity can be observed), predominantly inattentive type, and predominantly hyperactive-impulsive type.

According to National Institutes for Mental Health (NIMH), attention deficit/hyperactivity disorder is one of the most common psychiatric disorders in children. In U.S., approximately 4 millions of children have been diagnosed with attention deficit/hyperactivity disorder. The prevalence rate of attention deficit/hyperactivity disorder in school aged children is referred to be 3-7% (American Psychiatric Association: Diagnostic and Statistical Manual of Mental Disorders, 4th ed., Text Revision: DSM-IV-TR. American Psychiatric Association, Washington, D. C., 2000), and it is not small. Children with this disorder represent impairment in functioning within various environments, including relationship with family, school, and friends. Without treatment, attention deficit/hyperactivity disorder ill affects over a long period from childhood to adulthood.

Hyperactivity can be generally improved as one grows older, and therefore, attention deficit/hyperactivity disorder had been thought to be a disease peculiar to children. However, the thought has been recently changed, and it was found out that attention deficit/hyperactivity disorder is present also in adult. From the literature of Weiss, M. *et al.* which showed that the prevalence rate in school aged children was presumed to be 3 to 10%, and 30 to 50% of those individuals continued to have the disorder into adulthood (Weiss, M., Murray, C., Can. Med. Assoc. J., 168: 715-722, 2003), Clarke, S. *et al.* discloses that 1 to 5% of the adult population have symptoms of attention deficit/hyperactivity disorder (Clarke, S., Heussler, H., Kohn, M. R., Intern. Med. J. 35: 721-725, 2005). Therefore, attention deficit/hyperactivity disorder has already become a disease not only peculiar to children.

Treatment of attention deficit/hyperactivity disorder utilizes mostly pharmacotherapy and psychosocial therapy. Pharmacotherapy is very effective in controlling symptoms, and causes practical changes in behavior or thinking, learning ability, and personal relationship of the patient. Among therapeutic agents, methylphenidate, a psychostimulant, is the first choice. Examples of symptoms on which methylphenidate works include hyperactivity, difficulty focusing attention, impulsivity, anxiety and the like. It was reported that methylphenidate relieves 70 to 80% of symptoms of children with attention deficit/hyperactivity disorder (Non-patent Document 1), while methylphenidate has a risk of abuse because of the nature of the psychostimulant. Therefore, methylphenidate is classified as a Schedule II controlled substance, the designation used for substances that present a high likelihood for abuse in U.S. It is also a Schedule II drug under the Convention on Psychotropic Substances, and in Japan, it is also a first-class psychotropic drug and its use is being strictly limited.

Potassium channels are proteins which are present in the plasma membrane of cells and selectively pass potassium ions, and are considered to play an important role for control of membrane potential in cells. In particular, potassium channels contribute to the neurotransmission mechanism of central and peripheral nerves, heart pace-making, contraction of muscles and the like, by regulating frequency, durability and the like of the action potential in neurons and muscle cells. It is also known that potassium channels are involved in secretion of hormones, adjustment of cell capacity, cell proliferation, and the like.

According to the classification based on the gating mechanism, and so far, voltage-dependent potassium channels, inwardly rectifying potassium channels, calcium-dependent potassium channels, receptor-coupled potassium channels and the like have been identified. Among these, the voltage-dependent potassium channels are characterized by being opened when the membrane potential is depolarized. Typically, potassium ions exist in a non-equilibrium state of about 5 mM in the extracellular part and about 150 mM in the intracellular part. For this reason, when the voltage-dependent potassium channels are opened due to depolarization, potassium ions are discharged from the intracellular part to the extracellular part, and consequently induce recovery (repolarization) of the membrane potential. Therefore, a decrease in the excitability of neurons and muscle cells is induced, concomitantly with the opening of the voltage-dependent channels (Hille, B. (ed) Ionic Channels of Excited Membranes, Sinauer Associates, Sunderland, 1992).

A compound modifying the opening of the voltage-dependent channels regulates various physiological phenomena by regulating the excitability of neurons, muscle cells and the like, and also has a possibility of serving as a therapeutic agent for various diseases. For example, 4-aminopyridine which is an inhibitor of A-type voltage-dependent potassium channels found in nerve cells is known to induce epilepsy by increasing the nerve excitability (Yamaguchi, S. and Rogawski, M. A., Epilepsy Res. 11: 9-16, 1992). Further, dofetilide which is an inhibitor of hERG potassium channels expressed in the heart among the voltage-dependent potassium channels, is used as a drug for treatment arrhythmia based on the controlling of the excitability of myocardial cells (Gwilt, M., Arrowsmith, J. E., Blackburn, K. J., Burges, R. A., Cross, P. E., Dalrymple, H. W. and Higgins, A. J. J.Pharmacol. Exp. Ther. 256: 318-324, 1991).

The potassium channel as set forth in SEQ ID NO:2 in Example 1 of Patent Document 1 (hereinafter, referred to as BEC1 or BEC1 potassium channel) is a voltage-dependent potassium channel of which expression exhibits only a limited distribution in the brain. Expression of this channel is conspicuous in the hippocampus or the cerebral cortex. The hippocampus and cerebral cortex are regions suggested to be strongly associated with learning and memory (Levitan, I. B. and Kaczmarek, L. K. The neuron: Cell and Molecular Biology, Oxford University Press, New York, NY, 1991).

From this, the possibility that the BEC1 potassium channel is associated with learning and memory can be conceived. In fact, it was revealed with regard to a transgenic mouse having the BEC1 channel over-expressed in the hippocampus and the cerebral cortex, that the mouse has a decreased learning ability in the Morris water maze learning test and the passive avoidance learning test (Patent Document 2). From this fact, it is conceived that an inhibitor of BEC1 potassium channels enhances learning and memory, and thus is considered to be highly promising as a therapeutic agent for dementia.

A number of potassium channel inhibitors have been reported hitherto, but compounds that inhibit a BEC1 potassium channel are barely reported. While tetraethylammonium or quinidine is known to inhibit many potassium channels, both of them do not inhibit BEC1 potassium channels and only the 2,4,6-triamino-1,3,5-triazine derivatives are reported to inhibit BEC1 potassium channels (Patent Document 3). In this report, test results of the BEC1 channel inhibitor containing 2,4,6-triamino-1,3,5-triazine derivative on learning impairment are disclosed and that the BEC1 channel inhibitor is useful as agent for treating dementia is also disclosed. Meanwhile, there has been no report to date on a finding suggesting that the BEC1 channel inhibitors show usefulness for attention deficit/hyperactivity disorder.

### Related Art

### Patent Document

Patent Document 1: U.S. Patent No. 6,326,168
Patent Document 2: U.S. Patent No. 7,094,948
Patent Document 3: U.S. Patent No. 7,375,222

### Non-Patent Document

Non-patent Document 1: Kutcher, S., Aman, M., Brooks, S.J., Buitelaar, J., van Daalen, E., Fegert, J., Findling, R.L., Fisman, S., Greenhill, L.L., Huss, M., Kusumakar, V., Pine, D., Taylor, E. and Tyano, S., Eur. Neuropsychopharmacol., 14: 11-28, 2004

### Disclosure of Invention

### Problems to Be Solved by the Invention

The present invention is to provide a therapeutic agent for attention deficit/hyperactivity disorder having a novel mechanism of action which is different from conventional psychostimulants.

### Means for Solving the Problems

In order to address the above-mentioned technical problem, the inventors conducted research based on a unique idea, and found that BEC1 potassium channel inhibitors exhibit a remarkable therapeutic effect on attention deficit/hyperactivity disorder, thereby completing the present invention.

An object of the present invention is to provide a pharmaceutical composition for prevention and/or treatment of attention deficit/hyperactivity disorder, comprising 1) a BEC1 potassium channel inhibitor or a pharmaceutically acceptable salt thereof, and 2) a pharmaceutically acceptable carrier.
Another object of the present invention is to provide a prophylactic and/or therapeutic agent for attention deficit/hyperactivity disorder, comprising a BEC1 potassium channel inhibitor or a pharmaceutically acceptable salt thereof as an active ingredient.
Still another object of the present invention is to provide a BEC1 potassium channel inhibitor or a pharmaceutically acceptable salt thereof for prevention and/or treatment of attention deficit/hyperactivity disorder.
Still another object of the present invention is to provide a use of a BEC1 potassium channel inhibitor or a pharmaceutically acceptable salt thereof for manufacture of a medicament for treating attention deficit/hyperactivity disorder.
Still another object of the present invention is to provide a method of treating attention deficit/hyperactivity disorder, comprising administering an effective dose of a BEC1 potassium channel inhibitor or a pharmaceutically acceptable salt thereof to a patient.
Still another object of the present invention is to provide a method for preparing a pharmaceutical composition for treating attention deficit/hyperactivity disorder, the method comprising mixing a BEC1 potassium channel inhibitor or a pharmaceutically acceptable salt thereof, and a pharmaceutically acceptable excipient.
Still another object of the present invention is to provide a commercial package comprising a pharmaceutical composition comprising a BEC1 potassium channel inhibitor or a pharmaceutically acceptable salt thereof as an active ingredient, and an instructions describing that the BEC1 potassium channel inhibitor or a pharmaceutically acceptable salt thereof can be used or should be used to treat attention deficit/hyperactivity disorder.

### Effects of the Invention

The present invention is useful for providing a pharmaceutical composition for prevention and/or treatment of attention deficit/hyperactivity disorder, comprising 1) a BEC1 potassium channel inhibitor or a pharmaceutically acceptable salt thereof, and 2) a pharmaceutically acceptable carrier. Particularly, the present invention is useful for providing a pharmaceutical composition for prevention and/or treatment of inattention, impulsivity, hyperactivity, and the like of attention deficit/hyperactivity disorder.

### Embodiments for Carrying Out the Invention

Hereinafter, the present invention will be described in detail.

The term "hydrocarbon group" means linear or branched hydrocarbon group having 1 to 15 carbon atoms, preferably 1 to 10 carbon atoms, or cyclic hydrocarbon group having 3 to 15 carbon atoms. Linear or branched hydrocarbon group includes "alkyl", "alkenyl", "alkynyl" and the like. Specific examples of "alkyl" include methyl, ethyl, isopropyl, hexyl, decyl, tetradecyl, pentadecyl and the like. The term "alkenyl" means hydrocarbon group having at least one double bond, and specific examples of "alkenyl" include vinyl, propenyl, allyl, isopropenyl, hexenyl and the like. The term "alkynyl" means hydrocarbon group having at least one triple bond, and specific examples of "alkynyl" include ethynyl, propynyl, butynyl and the like. Cyclic hydrocarbon group includes "cycloalkyl", "cycloalkenyl", "aryl" and the like.

The term "lower alkyl" means linear or branched alkyl having 1 to 6 carbon atoms (hereinafter, abbreviated to C₁₋₆), and includes, for example, methyl, ethyl, n-propyl, isopropyl, n-butyl, isobutyl, sec-butyl, tert-butyl, n-pentyl, n-hexyl groups, and the like. In another embodiment, the lower alkyl is C₁₋₄ alkyl, and in still another embodiment, the lower alkyl is methyl, ethyl, propyl, isopropylbutyl, isobutyl, tert-butyl, pentyl or hexyl.

The term "C₁₋₁₀ alkylene" means linear or branched C₁₋₁₀ alkylene, and includes, for example, methylene, ethylene, trimethylene, tetramethylene, pentamethylene, hexamethylene, heptamethylene, octamethylene, nonamethylene, decamethylene, propylene, methylmethylene, ethylethylene, 1,2-dimethylethylene, 1,1,2,2-tetramethylethylene and the like.

The term "halogen" means F, Cl, Br or I.

The term "cycloalkyl" means a C₃₋₁₅ saturated hydrocarbon cyclic group, and may be bridged. The cycloalkyl includes, for example, cyclopropyl, cyclobutyl, cyclopentyl, cyclohexyl, cycloheptyl, cyclooctyl, adamantyl groups and the like. In another embodiment, the cycloalkyl is C₃₋₁₅ saturated hydrocarbon cyclic group, and in still another embodiment, the cycloalkyl is C₃₋₈ cycloalkyl, and in still another embodiment, the cycloalkyl is C₃₋₆ cycloalkyl.

The term "cycloalkenyl" means a C₄₋₁₅ cycloalkenyl, and may be bridged, and includes a condensed cyclic group with a benzene ring at a double bond site. The cycloalkenyl includes, for example, cyclopentenyl, cyclopentadienyl, cyclohexenyl, cyclohexadienyl, 1-tetrahydronaphtyl, 1-indenyl, 9-fluorenyl, and the like. In another embodiment, the cycloalkenyl is C₅₋₁₀ Cycloalkenyl.

The term "aryl" means a C₆₋₁₄ monocyclic to tricyclic aromatic hydrocarbon cyclic group, and includes, for example, phenyl and naphthyl. In another embodiment, the aryl is phenyl.

The term "heterocyclic" group means a 3- to 15-membered, in another embodiment, 5- to 10-membered, monocyclic to tricyclic heterocyclic group containing 1 to 4 heteroatoms selected from oxygen, sulfur and nitrogen, and includes a saturated cyclic group, an aromatic cyclic group, and a partially hydrogenated cyclic group. The sulfur or nitrogen atom, both of which are ring atoms, may be oxidized to form oxide or dioxide. Specific examples include monocyclic heteroaryl such as pyridyl, pyrrolyl, pyrazinyl, pyrimidinyl, pyridazinyl, imidazolyl, triazolyl, triazinyl, tetrazolyl, thiazolyl, pyrazolyl, isothiazolyl, oxazolyl, isoxazolyl, thiadiazolyl, oxadiazolyl, thienyl, or furyl; bicyclic heteroaryl such as indolyl, isoindolyl, benzimidazolyl, indazolyl, quinolyl, isoquinolyl, quinazolinyl, quinoxalinyl, phthalazinyl, benzothiazolyl, benzisothiazolyl, benzothiadiazolyl, benzoxazolyl, benzisoxazolyl, benzofuranyl or benzothienyl; tricyclic heteroaryl such as carbazolyl, dibenzo[b,d]furanyl or dibenzo[b,d]thienyl; non-aromatic monocyclic heterocyclic ring such as azetidinyl, pyrrolidinyl, piperidyl, piperazinyl, azepanyl, diazepanyl, morpholinyl, thiomorpholinyl, tetrahydropyridinyl, oxetanyl, tetrahydrofuranyl, tetrahydropyranyl, dioxolanyl, dioxanyl or tetrahydrothiopyranyl; non-aromatic bicyclic heterocyclic ring such as indolinyl, tetrahydroquinolyl, tetrahydroisoquinolyl, dihydrobenzimidazolyl, tetrahydrobenzimidazolyl, tetrahydroquinoxalinyl, dihydroquinoxalinyl, dihydrobenzoxazolyl, dihydrobenzoxazinyl, dihydrobenzofuryl, chromanyl, chromenyl, methylenedioxyphenyl or ethylenedioxyphenyl; bridged heterocyclic rings such as quinuclidinyl; and the like. In another embodiment, the heterocyclic group is a 5- to 10-membered monocyclic or bicyclic heterocyclic group, and in still another embodiment, the heterocyclic group is a 5-to 6-membered monocyclic heterocyclic group, and in still another embodiment, the heterocyclic group is 5- to 6-membered monocyclic heteroaryl.

In the present specification, the term "may be substituted" means "is not substituted" or "is substituted with 1 to 5 substituents". In case of having a plurality of substituents, these substituents may be the same or different from one another.

The substituent of "hydrocarbon group" and "heterocyclic group" of "hydrocarbon group which may be substituted" and "heterocyclic group which may be substituted" is group selected from the following Group A.
Group A
   -OH, -NH₂, -NH(lower alkyl), -N(lower alkyl)₂, -CN, -COOH, NO₂ lower alkyl, lower alkyl-O-, halogen, cycloalkyl, aryl and a heterocyclic group
   Here, the cycloalkyl, aryl and heterocyclic group described in the Group A may be substituted with a substituent selected from the following Group B.
Group B
   -OH, -NH₂, -NH(lower alkyl), -N(lower alkyl)₂, -CN, -COOH, NO₂ lower alkyl, lower alkyl-O-, halogen, cycloalkyl, aryl and a heterocyclic group

The term "BEC1" or "BEC1 potassium channel" means the full-length protein as set forth in SEQ ID NO:2 in U.S. Patent No. 6,326,168 or U.S. Patent No. 7,375,222.

The term "BEC1 potassium channel inhibitor" means a substance inhibiting the BEC1 potassium channel, and for example, it means a substance having an IC₅₀ value of 10 µM or less; in another embodiment, 1 µM or less; and in still another embodiment, 0.5 µM or less, based on the evaluation method described later in Example 1. The BEC1 potassium channel inhibitory activity can be measured by subjecting a test compound to a representative screening method, for example, the method described in U.S. Patent No. 6,326,168 or U.S. Patent No. 7,375,222.

Preferred embodiments of the present invention will be presented in the following.
(1) A pharmaceutical composition for prevention and/or treatment of attention deficit/hyperactivity disorder, wherein the BEC1 potassium channel inhibitor is a compound of the following formula (I): 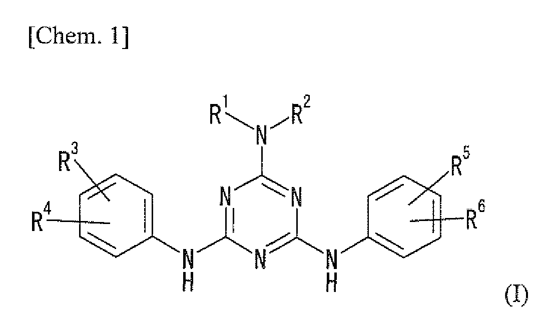 wherein the symbols are as follows;
   each of R¹ and R², which may be the same or different, represents (i) H, (ii) OH, (iii) alkyl-O-, (iv) aryl-CO-, (v) H₂N, (vi) (alkyl which may be substituted with OH)-NH, (vii) (alkyl)₂N, (viii) a hydrocarbon group which may be substituted, or (ix) a heterocyclic group which may be substituted; and
   each of R³, R⁴, R⁵ and R⁶, which may be the same or different, represents (i) H, (ii) CN, (iii) NO₂ (iv) halogen, (v) lower alkyl which may be substituted with a group selected from the group consisting of CN, halogen, and OH, (vi) cycloalkyl, (vii) aryl which may be substituted with lower alkyl, (viii) a heterocyclic group which may be substituted with lower alkyl, (ix) R⁷R⁸N- (wherein each of R⁷ and R⁸ may be the same or different, and represents (1) H, (2) lower alkyl which may be substituted with a group selected from the group consisting of aryl and R⁹-O-CO- (wherein R⁹ represents (1) H, or (2) lower alkyl which may be substituted with aryl)), (x) R¹⁰-T¹- (wherein R¹⁰ represents (1) H, (2) lower alkyl which may be substituted with a group selected from the group consisting of aryl, HO-C₁₋₁₀ alkylene-O- and HO, or (3) aryl; and T¹ represents O or S), or (xi) R¹¹-T²-(wherein R¹¹ represents (1) OH, (2) R⁷R⁸N-, (3) lower alkyl-O-, (4) lower alkyl, (5) aryl, or (6) a heterocyclic group; and T² represents CO or SO₂).
(2) The pharmaceutical composition according to (1), wherein each of R¹ and R², which may be the same or different, represents (i) H, or (ii) lower alkyl which may be substituted with one heterocyclic group which may be substituted; and each of R³, R⁴, R⁵ and R⁶, which may be the same or different, represents (i) H, (ii) halogen, or (iii) lower alkyl-O-.
(3) The pharmaceutical composition according to (1) or (2), wherein each of R¹ and R², which may be the same or different, represents (i) H, or (ii) lower alkyl which may be substituted with one heterocyclic group selected from pyrimidine and pyridine, which may be substituted with a group selected from the group consisting of halogen, lower alkyl and lower alkyl-O-.
(4) The pharmaceutical composition according to (3), wherein R¹ represents H;
   R² represents lower alkyl substituted with one heterocyclic group selected from pyrimidine and pyridine, which may be substituted with a group selected from the group consisting of halogen, lower alkyl and lower alkyl-O-;
   each of R³ and R⁶ represents H; and
   each of R⁴ and R⁵, which may be the same or different, represents (i) H, (ii) halogen, or (iii) lower alkyl-O-.
(5) The pharmaceutical composition according to (4), wherein R¹ represents H;
   R² represents lower alkyl substituted with pyrimidine which may be substituted with a group selected from the group consisting of halogen, lower alkyl and lower alkyl-O-;
   each of R³ and R⁶ represents H; and
   each of R⁴ and R⁵, which may be the same or different, represents (i) H, (ii) halogen, or (iii) lower alkyl-O-.
(6) The pharmaceutical composition according to (4), wherein R¹ represents H;
   R² represents lower alkyl substituted with pyridine which may be substituted with a group selected from the group consisting of halogen, lower alkyl and lower alkyl-O-;
   each of R³ and R⁶ represents H; and
   each of R⁴ and R⁵, which may be the same or different, represents (i) H, (ii) halogen, or (iii) lower alkyl-O-.
(7) The pharmaceutical composition according to (1) to (6), wherein the attention deficit/hyperactivity disorder is inattention, impulsivity, and hyperactivity in attention deficit/hyperactivity disorder.

Specific examples of compounds of the formula (I) included in the present invention include the following compounds:
N-(4-fluorophenyl)-N'-phenyl-N"-(pyrimidin-2-ylmethyl)-1,3,5-triazine-2,4,6-triamine, N,N'-bis(4-fluorophenyl)-N"-(pyrimidin-2-ylmethyl)-1,3,5-triazine-2,4,6-triamine, N-(4-fluorophenyl)-N'-(4-methoxyphenyl)-N"-(pyrimidin-2-ylmethyl)-1,3,5-triazine-2,4,6-triamine, N,N'-bis(4-fluorophenyl)-N"-(pyrimidin-4-ylmethyl)-1,3,5-triazine-2,4,6-triamine, or N-(4-fluorophenyl)-N'-[(2-fluoro-4-pyridyl)methyl]-N"-phenyl-1,3,5-triazine-2,4,6-triamine.

The compound of the formula (I) may have tautomers or geometric isomers, depending on the type of substituent. In the present specification, the compound of the formula (I) may be described only as one form of isomers in some cases, but the present invention also includes the other isomers, as well as separated isomers or mixtures thereof. The compound of the formula (I) may also have asymmetric carbon atoms or axial asymmetry, and optical isomers based thereon may also exist. The present invention includes separated optical isomers of the compound of the formula (I), or mixtures thereof,

The present invention also includes pharmaceutically acceptable prodrugs of the compound represented by the formula (I). A pharmaceutically acceptable prodrug is a compound having a group which can be converted to the amino group, hydroxyl group, carboxyl group, or the like (of the present invention) by solvolysis or under physiological conditions. Examples of the group forming a prodrug include groups described in Prog. Med., 5, 2157-2161 (1985) or "Pharmaceutical Research and Development" (Hirokawa Publishing Company, 1990), Vol. 7 Drug Design, 163-198.

The compound of the formula (I) may also form an acid addition salt or a salt with a base depending on the type of substituent, and such salt is included in the present invention so long as it is a pharmaceutically acceptable salt. Specific examples include acid addition salts with an inorganic acid such as hydrochloric acid, hydrobromic acid, hydroiodic acid, sulfuric acid, nitric acid, phosphoric acid or the like; acid addition salts with an organic acid such as formic acid, acetic acid, propionic acid, oxalic acid, malonic acid, citric acid, fumaric acid, maleic acid, lactic acid, malic acid, mandelic acid, tartaric acid, dibenzoyltartaric acid, ditoluoyltartaric acid, citric acid, methanesulfonic acid, ethanesulfonic acid, benzenesulfonic acid, p-toluenesulfonic acid, aspartic acid, glutamic acid or the like; salts with an inorganic base such as sodium, potassium, magnesium, calcium, aluminum or the like; salts with an organic base such as methylamine, ethylamine, ethanolamine, lysine, ornithine, or the like; salts with various amino acids such as acetylleucine or the like, and amino acid derivatives; ammonium salts; or the like.

The compound of the formula (I) and/or pharmaceutically acceptable salts thereof can be obtained by the production method described in U.S. Patent No. 7,375,222, or by a production method equivalent thereto.

A pharmaceutical composition containing one or two or more of the compound of the formula (I), or one or two or more of pharmaceutically acceptable salts thereof, as an active ingredient can be prepared by using pharmaceutical excipients, pharmaceutical carriers and the like that are conventionally used in the pertinent art, according to a conventionally used method.
Administration may be carried out by any of the oral administration mode by means of tablets, pills, capsules, granules, powders, liquids or the like, or the parenteral administration mode by means of injectable preparations via intraarticular, intravenous, intramuscular routes, suppositories, eye drops, eye ointments, transdermal liquids, ointments, transdermal adhesive patches, transmucosal liquids, transmucosal adhesive patches, inhalants or the like.

As solid compositions for oral administration, tablets, powders, granules and the like are used. In these solid compositions, one or two or more active ingredients are mixed with at least one inert excipient, for example, lactose, mannitol, glucose, hydroxypropylcellulose, microcrystalline cellulose, starch, polyvinylpyrrolidone, and/or magnesium metasilicate aluminate and the like. The composition may contain inert additives, for example, a gliding agent such as magnesium stearate, a disintegrant such as carboxymethyl starch sodium, a stabilizer, and a dissolution aid, according to standard methods. Tablets or pills may be coated, if necessary, with sugar coating or a film of a gastrosoluble or enterosoluble material.
Liquid compositions for oral administration include pharmaceutically acceptable emulsions, solutions, suspensions, syrups or elixirs and the like, and include a generally used inert diluent, for example, purified water or ethanol. The liquid compositions may also contain, in addition to the inert diluent, an auxiliary agent such as a solubilizer, a wetting agent, and a suspending agent, a sweetener, a flavor, an aromatic or an antiseptic.

An injectable preparation for parenteral administration contains a sterile, aqueous or non-aqueous solution, suspension or emulsion. Examples of aqueous solvents include distilled water for injection or physiological saline. Examples of non-aqueous solvents include propylene glycol, polyethylene glycol, plant oils such as olive oil, alcohols such as ethanol, Polysorbate 80 (name in the Japanese Pharmacopoeia), and the like. These compositions may further include an isotonic agent, an antiseptic, a wetting agent, an emulsifier, a dispersant, a stabilizer or a dissolution aid. These are sterilized by, for example, filtration through a bacteria-retaining filter, incorporation of a bactericide, or irradiation. Further, these can be used such that a sterile solid composition is prepared, and then dissolved or suspended in sterilized water or in a sterile solvent for injection before use.

Topical preparations include ointments, plasters, creams, jellies, adhesive skin patches, sprays, lotions, eye drops, eye ointments and the like. The topical preparations contain generally used ointment bases, lotion bases, aqueous or non-aqueous liquids, suspensions, emulsions and the like. Examples of the ointment or lotion base include polyethylene glycol, propylene glycol, white petrolatum, bleached beeswax, polyoxyethylene hydrogenated castor oil, glycerin monostearate, stearyl alcohol, cetyl alcohol, lauromacrogol, sorbitan sesquioleate and the like.

The preparations for transmucous application such as inhalants or preparations for transnasal application are used in a solid, liquid or semi-solid form, and can be produced according to conventionally known methods. For example, known excipients, and further, a pH adjusting agent, an antiseptic, a surfactant, a gliding agent, a stabilizer or thickening agent, and the like may be appropriately added. Administration can be carried out by using appropriate devices for inhalation or insufflation. For example, the compound can be administered alone or as a powder of a prescribed mixture, or as a solution or suspension in combination with a pharmaceutically acceptable carrier, using a known device such as a metered dose inhaler, or a sprayer. A dry powder inhaler or the like may be for a single dose or multiple doses, and dry powders or powder-containing capsules can be used. Alternatively, the preparations may also be in the form of an appropriate ejector, for example, a pressurized aerosol spray using a suitable gas such as chlorofluoroalkane, hydrofluoroalkane or carbon dioxide.

Typically, in the case of oral administration, the daily dosage is appropriately about 0.001 to 100 mg/kg, preferably 0.1 to 30 mg/kg, and more preferably 0.1 to 10 mg/kg, of body weight, and this is administered once, or in two to four divided portions. In the case of intravenous administration, the daily dosage is appropriately about 0.0001 to 10 mg/kg of body weight, and this is administered once or in many divided portions per day. As for the preparations transmucous application, about 0.001 to 100 mg/kg of body weight is administered once or in multi-divided portions per day. The dosage is appropriately determined in accordance with the individuals, while taking symptoms, age, gender, and the like into consideration.

The compound of the formula (I) can be used in combination with an agent for treating or preventing attention deficit/hyperactivity disorder. This combination may be administered simultaneously, or separately and sequentially, or even may be administered at a desired time interval. The preparation for simultaneous administration may be a blend preparation, or may be separately formulated.

### Examples

The following Reference Examples and Examples are intended to describe the present invention in more detail, and the present invention is not limited to the following Examples. Although the present invention is sufficiently described by the Reference Examples and Examples, those ordinarily skilled in the art will understand that various alterations or modifications are definitely possible. Therefore, as long as such alterations or modifications do not depart from the scope of the present invention, they are included in the present invention.

In the Reference examples, examples and tables described below, the following abbreviations will be used.
REx: Reference example number, mp: melting point, DATA: Physicochemical data (FAB+: FAB-MS(M+H)⁺, EI: EI-MS(M)⁺, NMR-DMSOd6: δ (ppm) of peaks from ¹H NMR in DMSO-d₆), DMSO: dimethylsulfoxide, THF: tetrahydrofuran, 4 M hydrogen chloride/dioxane solution: 4 mol/L hydrogen chloride dioxane solution, MeCN: acetonitrile, MeOH: methanol, EtOH: ethanol.

### Reference Example 1-1

75.0 g of cyanuric chloride and 680 ml of THF were added to a 2-L flask, followed by addition of 51.10 g of potassium carbonate at -19°C with stirring. 41.08 g of p-fluoroaniline that has been diluted with 75 ml of THF at -12.4°C or lower, and 75 ml of THF were added thereto. The reaction was carried out at -12.8 to -14.4°C for 1 hour, and 450 ml of water was added. Liquid separation was carried out at room temperature to separate the aqueous layer, 300 ml of water was added thereto, and liquid separation was carried out again to separate the aqueous layer. To the organic layer were added 600 ml of THF and an aqueous solution obtained by adding 1.1 g of potassium carbonate to 308 ml of water, and liquid separation was carried out to separate the aqueous layer. To the organic layer was added 150 ml of water, liquid separation was carried out to separate the aqueous layer, and the organic layer was concentrated under reduced pressure until the remaining amount of the solution became 280 ml. To the concentrated solution was added 750 ml of MeCN, and the concentration operation was carried out three times under reduced pressure until the remaining amount of the solution became 280 ml. Subsequently, 600 ml of MeCN was added thereto. After cooling, 34.43 g of aniline and 75 ml of MeCN were added at -5.9°C or less, and 47.79 g of N,N-diisopropylethylamine and 38 ml of MeCN was added at -9.2°C. Thereafter, the temperature was elevated to room temperature, and after stirring for 12 hours, 48.42 g of 2-aminomethylpyrimidine and 75 ml of MeCN were added thereto at room temperature, followed by addition of 57.35 g of N,N-diisopropylethylamine and 38 ml of MeCN at room temperature. The inner temperature was elevated to 82.4°C, followed by stirring for 4.5 hours, and 560 ml of water was added thereto at an inner temperature of 70°C or higher, followed by cooling. The crystal precipitation at an inner temperature of 65.8°C was confirmed, followed by standing at room temperature overnight, and filtration. The obtained crystal was washed with a mixed solution of MeCN:water = 2:1, and subsequently washed with 300 ml of water. The obtained crystal was dried at 50°C for 1 day under reduced pressure to obtain 108.54 g of N-(4-fluorophenyl)-N'-phenyl-N"-(pyrimidin-2-ylmethyl)-1,3,5-triazine-2,4,6-triamine.

### Reference Example 1-2

414 L of methyl ethyl ketone and 23.00 kg of the crystal of N-(4-fluorophenyl)-N'-phenyl-N"-(pyrimidin-2-ylmethyl)-1,3,5-triazine-2,4,6-triamine were added to a reaction vessel 1, and dissolved at an inner temperature of 65.0°C, followed by filtration, transferring to a reaction vessel 2, washing with 23 L of methyl ethyl ketone and then heating again. Separately, 6.90 kg of fumaric acid and 115 L of EtOH were added to the reaction vessel 1, dissolved at an inner temperature of 58.3°C, transferred to the reaction vessel 2, followed by washing with 23 L of EtOH. After cooling started, precipitation of crystals at an inner temperature of 54.2°C was confirmed, followed by stirring at 0°C overnight. The crystal was collected by filtration and washed with 46 L of EtOH, and 30.34 kg (wet weight) of the obtained "crystal of the salt having a ratio of the N-(4-fluorophenyl)-N'-phenyl-N"-(pyrimidin-2-ylmethyl)-1,3,5-triazine-2,4,6-triamine to fumaric acid of 1:1" and 460 L of EtOH were added to the reaction vessel 2. They were stirred at an inner temperature of 52.4 to 69.2°C in a suspension state for 42 hours, cooled, and stirred at room temperature overnight. The crystal was collected by filtration and washed with 46 L of EtOH, and then dried at 60°C for 4 days under reduced pressure to obtain 20.97 kg of a "crystal of an anhydrous salt having a ratio of the N-(4-fluorophenyl)-N'-phenyl-N"-(pyrimidin-2-ylmethyl)-1,3,5-triazine-2,4,6-triamine to fumaric acid of 2:1".

### Reference Example 2-1

To a mixed solution of 25 g of 2-pyrimidinecarbonitrile in 100 ml of acetic acid and 100 ml of ethyl acetate, 1 g of 10% palladium/carbon was added, followed by stirring at room temperature for 14 hours in a hydrogen atmosphere at room pressure. The palladium/carbon was removed from the reaction mixture by filtration through Celite, and the operations of distillation of the solvent, addition of toluene to the obtained residue, and concentration were repeated four times. MeCN was added to the obtained residue to solidification, and solids were collected by filtration, to obtain 15.7 g of 1-pyrimidin-2-ylmethylamine acetate as a colorless solid.
NMR-DMSOd6: 1.88 (3H, s), 3.91 (2H, brs), 4.1-5.3 (3H, m), 7.38 (1H,t, J=4.9Hz), 8.78 (2H, d, J=4.9Hz)
EI: 109

### Reference Example 2-2

To a solution of 4.71 g of 6-chloro-N,N'-bis(4-fluorophenyl)-1,3,5-triazine-2,4-diamine in 50 ml of MeCN, 2.507 g of 1-pyrimidin-2-ylmethylamine acetate and 5.2 ml of N,N-diisopropylethylamine were added, followed by stirring for 17 hours at 75°C. The reaction liquid was cooled to room temperature, and then to the residue obtained by distilling off the solvent, ethyl acetate was added. The organic layer was washed with 5% aqueous citric acid solution and saturated brine, and dried over anhydrous magnesium sulfate, and then the solvent was distilled off. The obtained residue was purified by silica gel column chromatography (chloroform:MeOH = 100:0 to 95:5), to obtain 6.0 g of a pale yellow amorphous material. This was dissolved in 180 ml of EtOH, 2 g of activated carbon was added thereto, followed by stirring for one hour. The activated carbon was removed by filtration through Celite, and the residue obtained by distilling off the solvent was solidified from 150 ml of aqueous EtOH (EtOH 80%), to obtain 4.84 g of N,N'-bis(4-fluorophenyl)-N"-(pyrimidin-2-ylmethyl)-1,3,5-triazine-2,4,6-triamine as a colorless solid.
1.5 g of the obtained N,N'-bis(4-fluorophenyl)-N"-(pyrimidin-2-ylmethyl)-1,3,5-triazine-2,4,6-triamine was dissolved in 300 ml of MeOH, and 2 ml of a 4 M hydrogen chloride/dioxane solution was added. Then, the solvent was distilled off, and the obtained residue was crystallized from ethanol, to obtain 1.66 g of a "salt of N,N'-bis(4-fluorophenyl)-N"-(pyrimidin-2-ylmethyl)-1,3,5-triazine-2,4,6-triamine and hydrogen chloride in a ratio of 1:2" as colorless crystals.

The compounds of Reference Example 3 ("salt of N-(4-fluorophenyl)-N'-(4-methoxyphenyl)-N"-(pyrimidin-2-ylmethyl)-1,3,5-triazinc-2,4,6-triamine and hydrogen chloride in a ratio of 1:2") and Reference Example 4 ("composition of N,N'-bis(4-fluorophenyl)-N"-(pyrimidin-4-ylmethyl)-1,3,5-triazine-2,4,6-triamme·1.7 hydrogen chloride·0.2 diethyl ether·1.8 H₂O") as shown in the following Table 2 were synthesized in the same manner as in Reference Examples 2-1 and 2-2.

The structures and property values of the Reference Example compounds are presented in the following Tables 1 and 2.

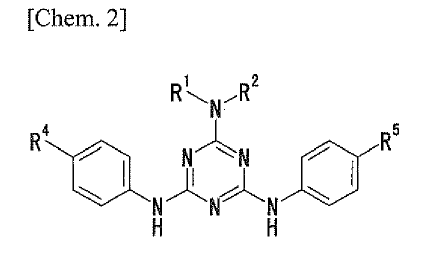

**[Table 1]**

| REx | 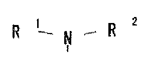 | R⁴ | R⁵ | Salt/ Solvent | DATA |
|---|---|---|---|---|---|
| 1 - 1 | 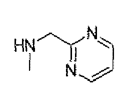 | F | H | - | NMR-DMSOd6 |
| | | | | | 4.71-4.73 (2H,m), 6.91-7.26 (5H,m), 7.37 (1H,dd, J=5.2Hz, 4.8Hz), 7.44-7.80 (5H,m), 8.78 (2H,d, J=4.8Hz), 9.01-9.05 (2H,m) |
| | | | | | FAB+: 389 |
| | | | | | Elemental Analysis. Calcd for C20H17FN8: C, 61.85; H, 4,41; N, 28.85; F, 4.89; Cl, 0.00. Fond: C, 61.78; H, 4.43; N, 28.81; F, 4.95; Cl, 0.00 |
| 1 - 2 | 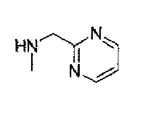 | F | H | 0.5 Fumaric acid | NMR-DMSOd6 |
| | | | | | 4.71-4.73 (2H,m), 6.64 (1H,s), 6.91-7.23 (5H,m), 7.37ppm (1H,dd, J=5.2Hz, 4.8Hz), 7.44-7.80 (5H,m), 8.78 (2H,d, J=4.8Hz), 9.01-9.06 (2H,m),13.06 (1H,br) |
| | | | | | FAB+ : 389 |
| | | | | | Elemental Analysis. Caled for C20H17FN8 · 0.5C4H4O4: C, 59.19; H, 4,29; N, 25.10; F, 4.26; O, 7.17. Found: C, 59.09; H, 4.36; N, 25.19; F, 4.31. |
| 2 - 2 | 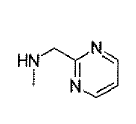 | F | F | 2HCl | NMR-DMSOd6 |
| | | | | | 4.78 (2H, m), 7.10 (2H, brs), 7.25 (2H, t, J=8.7Hz), 7.3-7.8 (6H, m), 8.85 (2H, d, J=4.9Hz), 8.9-9.4 (1H, m), 10.39 (1H, s), 10.74 (1H, brs) |
| | | | | | Elemental Analysis. Calcd for C20H16F2N8 · 2HCl: C, 50.12; H, 3.79; N, 23.38; F, 7.93; Cl, 14.79. Found: C, 50.06; H, 3.85; N, 23.38; F, 8.05; Cl, 14.91. |
| | | | | | mp: 183-186°C |

**[Table 2]**

| REx | 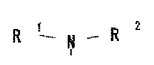 | R⁴ | R⁵ | Salt/ Solvent | DATA |
|---|---|---|---|---|---|
| 3 | 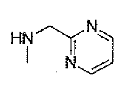 | F | OMe | 2HCl | NMR-DMSOd6 |
| | | | | | 3.70-3.82 (3H, m), 4.6-5.0 (2H, m), 6.7-7.8 (10H, m), 8.85 (2H, d, J=4.9Hz), 9.0-9.7 (1H, m), 10.1-11.3 (2H, m) |
| | | | | | FAB+ : 419 |
| 4 | 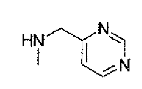 | F | F | 1.7HCl/ 0.2C₄H₁₀O / 1.8H₂O | NMR-DMSOd6 |
| | | | | | 4,65 (2H, brs), 6.8-7.3 (4H, m), 7.3-7.9 (6H, m), 8.6-9.0 (1H, m), 9.17 (1H, d, J=1.2Hz), 9.8-10.7 (2H, m) |
| | | | | | FAB+ : 407 |

### Example 1

### (Test Method)

### Method for measuring BEC1 inhibitory activity of compound utilizing 86Rb ion release amount as indicator

The channel activity of BEC1 was measured according to the method described in U.S. Patent No. 6,326,168 utilizing the release of the ions of radioactive isotope 86Rb from BEC1 expressing cells as an indicator. Specifically, when BEC1 expressing cells which had taken in 86Rb ions were stimulated with 100 mM KCl, the radioactivity released from the cells was designated as the channel activity of BEC1. 86Rb ions were incorporated into cells by culturing (3 hours, 37°C) BEC1 stably expressing cells in the presence of 86RbCl (0.5 µCi/ml), and the unincorporated 86Rb ions were removed by washing the cells three times with HEPES buffered physiological saline (pH 7.4, 2.5 mM KCl). The cells were incubated for 15 minutes at room temperature in the presence of a DMSO solution containing the test compound and HEPES buffered physiological saline, and then were further incubated for 5 minutes at room temperature in the presence of a 100 mM KCl-containing HEPES buffer solution (pH 7.4) containing the compound. The extracellular fluid was recovered, and then the remaining cells were lysed in 0.1 N NaOH and recovered.
The Cherenkov radioactivities of the extracellular fluid and the cell lysate were respectively measured, and the sum was designated as the total radioactivity. The release amount of 86Rb ions was expressed as the percentage of the radioactivity of the extracellular fluid to the total radiation activity. The value obtained in the presence of the compound was designated as a test value, the value obtained in the absence of the compound was designated as a control value, and the value obtained when the cells were not stimulated with 100 mM KCl was designated as a blank value. The inhibitory action of the compound was indicated as the IC₅₀ value determined from the inhibition % (that is, (control value-test value)×100/(control value-blank value)). Further, as for the BEC1 expressing cells, BEC1 stably expressing cells produced according to the method described in U.S. Patent No. 6,326,168, using a dihydrofolate reductase (dhfr)-deficient strain of Chinese Hamster ovary cells, were used.

### (Results)

The test results of representative compounds are presented in Table 3. The corresponding compounds were confirmed to have BEC1 potassium channel inhibitory action.

### Example 2

### (Experiment)

Verification of the therapeutic effect on attention deficit/hyperactivity disorder was carried out using spontaneously hypertensive stroke-prone rats (SHRSP). It was reported that this animal had the same properties with attention deficit/hyperactivity disorder patients such as an abnormality in the dopamine-incorporated protein and the like, and besides, showed hyperactivity, attention disorder, and impulsivity in early-life, and therefore, SHRSP might be an animal model of attention deficit/hyperactivity disorder (Ueno, K. et al., Behav. Pharmacol., 13: 1-13,2003). The method of Fox *et al.* (Fox, G. B. et al., Behav. Brain. Res., 131: 151-161, 2002) was partially modified, and the drug action on attention deficit/hyperactivity disorder was verified using a passive avoidance response test with SHRSP. That is, a solvent (vehicle), or dilutions prepared by diluting methylphenidate, compound (1), compound (2), compound (3), compound (4), and compound (5), with a solvent at multiple concentrations were administered to the rats in each group. Methylphenidate was subcutaneously administered, and compounds (1) to (5) were orally administered. As the solvent, physiological saline was used for methylphenidate and a 0.5% aqueous solution of methylcellulose was used for compounds (1) to (5). After 30 minutes of each drug administration, rats were placed in the bright room of the passive avoidance response measurement apparatus, and the time required for the rat to enter the dark room (entry latency) was measured. The longest time for measurement was 180 seconds. When rats entered in the dark room, a very weak electric current was forced to flow from a foot grid in the dark room. This trial was repeated five times, and the sum of the entry latency from 2nd time to 5th time (cumulative entry latency) was adopted as an indicator of the therapeutic effect on attention deficit/hyperactivity disorder. The test of significance difference was carried out between the solvent administered group and the drug administered groups, using Dunnett's test.

### (Results)

The results of five trials of passive avoidance response test with SHRSP are presented in Table 3. The numerical values in the table represent the respective minimum effective doses for the compound administered groups (the smallest dose inducing the significantly long cumulative entry latency of the drug administered group compared to the cumulative entry latency of the solvent administered group). Methylphenidate, compound (1), compound (2), compound (3), compound (4), and compound (5) all prolonged the cumulative entry latency of SHRSP. That is, it was found that compounds (1) to (5) exhibited the effect of improving symptoms of attention deficit/hyperactivity disorder as well as methylphenidate.

### Test Compounds

Compound (1) means the compound of REx 1-2, compound (2) means the compound of REx 2-2, compound (3) means the compound of REx 3, compound (4) means the compound of REx 4, and compound (5) means N-(4-fluorophenyl)-N'-[(2-fluoro-4-pyridyl)methyl]-N'-phenyl-1,3,5-triazine-2,4,6-triamine hydrochloride.

**[Table 3]**

| **Test Compound** | **BEC1 Inhibitory Activity IC₅₀(µM)** | **Minimum effective dose (mg/kg p.o.)** |
|---|---|---|
| (1) | **0.077** | **0.1** |
| (2) | **0.065** | **0.03** |
| (3) | **0.092** | **0.3** |
| (4) | **0.058** | **0.03** |
| (5) | **0.085** | **3** |
| **Methylphenidate** | **-** | **0.1(sc)** |

### Industrial Applicability

The present invention is useful for providing an excellent pharmaceutical composition for prevention and/or treatment of attention deficit/hyperactivity disorder, containing a BEC1 potassium channel inhibitor or a pharmaceutically acceptable salt thereof, and is particularly useful for providing a pharmaceutical composition for prevention and/or treatment of inattention, hyperactivity, and impulsivity of attention deficit/hyperactivity disorder.

## Claims

1. A pharmaceutical composition for prevention and/or treatment of attention deficit/hyperactivity disorder, comprising 1) a BEC1 potassium channel inhibitor or a pharmaceutically acceptable salt thereof, and 2) a pharmaceutically acceptable carrier.

2. The pharmaceutical composition according to claim 1, wherein the BEC1 potassium channel inhibitor is a compound of the following formula (I): 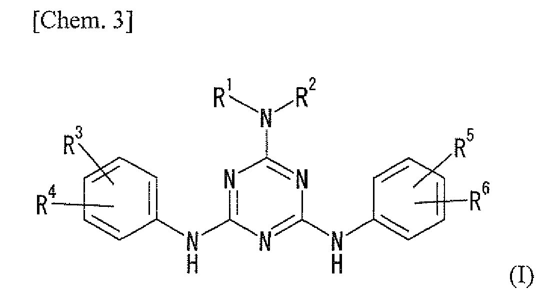 wherein the symbols are as follows;
each of R¹ and R², which may be the same or different, represents (i) H, (ii) OH, (iii) alkyl-O-, (iv) aryl-CO-, (v) H₂N, (vi) (alkyl which may be substituted with OH)-NH, (vii) (alkyl)₂N, (viii) a hydrocarbon group which may be substituted, or (ix) a heterocyclic group which may be substituted; and
each of R³, R⁴, R⁵ and R⁶, which may be the same or different, represents (i) H, (ii) CN, (iii) NO₂ (iv) halogen, (v) lower alkyl which may be substituted with a group selected from the group consisting of CN, halogen, and OH, (vi) cycloalkyl, (vii) aryl which may be substituted with lower alkyl, (viii) a heterocyclic group which may be substituted with lower alkyl, (ix) R⁷R⁸N- (wherein each of R⁷ and R⁸ may be the same or different, and represents (1) H, (2) lower alkyl which may be substituted with a group selected from the group consisting of aryl and R⁹-O-CO- (wherein R⁹ represents (1) H, or (2) lower alkyl which may be substituted with aryl)), (x) R¹⁰-T¹- (wherein R¹⁰ represents (1) H, (2) lower alkyl which may be substituted with a group selected from the group consisting of aryl, HO-C₁₋₁₀ alkylene-O- and HO, or (3) aryl; and T¹ represents O or S), or (xi) R¹¹-T²-(wherein R¹¹ represents (1) OH, (2) R⁷R⁸N-, (3) lower alkyl-O-, (4) lower alkyl, (5) aryl, or (6) a heterocyclic group; and T² represents CO or SO₂).

3. The pharmaceutical composition according to claim 2, wherein the formula (I) is a compound wherein
each of R¹ and R², which may be the same or different, represents (i) H or (ii) lower alkyl which may be substituted with one heterocyclic group which may be substituted; and
each of R³ R⁴, R⁵ and R⁶, which may be the same or different, represents (i) H, (ii) halogen, or (iii) lower alkyl-O-.

4. An prophylactic and/or therapeutic agent for attention deficit/hyperactivity disorder , comprising a BEC1 potassium channel inhibitor or a pharmaceutically acceptable salt thereof as an active ingredient.

5. The prophylactic and/or therapeutic agent according to claim 4, wherein the BEC1 potassium channel inhibitor is a compound of the following formula (I): 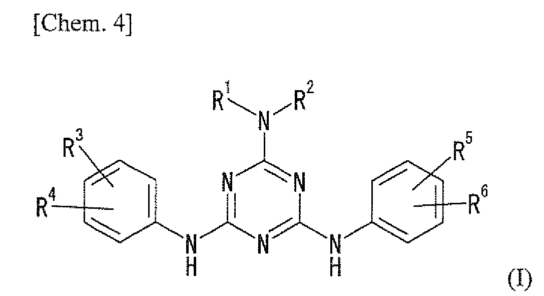 wherein the symbols are as follows;
each of R¹ and R², which may be the same or different, represents (i) H, (ii) OH, (iii) alkyl-O-, (iv) aryl-CO-, (v) H₂N, (vi) (alkyl which may be substituted with OH)-NH, (vii) (alkyl)₂N, (viii) a hydrocarbon group which may be substituted, or (ix) a heterocyclic group which may be substituted; and
each of R³, R⁴, R⁵ and R⁶, which may be the same or different, represents (i) H, (ii) CN, (iii) NO₂ (iv) halogen, (v) lower alkyl which may be substituted with a group selected from the group consisting of CN, halogen, and OH, (vi) cycloalkyl, (vii) aryl which may be substituted with lower alkyl, (viii) a heterocyclic group which may be substituted with lower alkyl, (ix) R⁷R⁸N- (wherein each of R⁷ and R⁸ may be the same or different, and represents (1) H, (2) lower alkyl which may be substituted with a group selected from the group consisting of aryl and R⁹-O-CO- (wherein R⁹ represents (1) H, or (2) lower alkyl which may be substituted with aryl)), (x) R¹⁰-T¹- (wherein R¹⁰ represents (1) H, (2) lower alkyl which may be substituted with a group selected from the group consisting of aryl, HO-C₁₋₁₀alkylene-O- and HO, or (3) aryl; and T¹ represents O or S), or (xi) R¹¹-T²-(wherein R¹¹ represents (1) OH, (2) R⁷R⁸N-, (3) lower alkyl-O-, (4) lower alkyl, (5) aryl, or (6) a heterocyclic group; and T² represents CO or SO₂).

6. The prophylactic and/or therapeutic agent according to claim 5, wherein the formula (1) is a compound wherein
each of R¹ and R², which may be the same or different, represents (i) H or (ii) lower alkyl which may be substituted with one heterocyclic group which may be substituted; and
each of R³, R⁴, R⁵ and R⁶, which may be the same or different, represents (i) H, (ii) halogen, or (iii) lower alkyl-O-.

7. A BEC1 potassium channel, inhibitor or a pharmaceutically acceptable salt thereof for prevention and/or treatment of attention deficit/hyperactivity disorder.

8. The compound or a pharmaceutically acceptable salt thereof according to claim 7, wherein the BEC1 potassium channel inhibitor is a compound of the following formula (I): 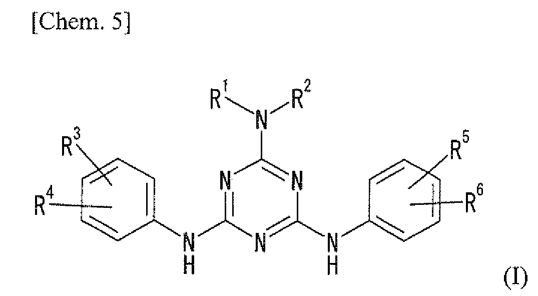 wherein the symbols are as follows;
each of R¹ and R², which may be the same or different, represents (i) H, (ii) OH, (iii) alkyl-O-, (iv) aryl-CO-, (v) H₂N, (vi) (alkyl which may be substituted with OH)-NH, (vii) (alkyl)₂N, (viii) a hydrocarbon group which may be substituted, or (ix) a heterocyclic group which may be substituted; and
each of R³, R⁴, R⁵ and R⁶, which may be the same or different, represents (i) H, (ii) CN, (iii) NO₂ (iv) halogen, (v) lower alkyl which may be substituted with a group selected from the group consisting of CN, halogen, and OH, (vi) cycloalkyl, (vii) aryl which may be substituted with lower alkyl, (viii) a heterocyclic group which may be substituted with lower alkyl, (ix) R⁷R⁸N- (wherein each of R⁷ and R⁸ may be the same or different, and represents (1) H, (2) lower alkyl which may be substituted with a group selected from the group consisting of aryl and R⁹-O-CO- (wherein R⁹ represents (1) H, or (2) lower alkyl which may be substituted with aryl)), (x) R¹⁰-T¹- (wherein R¹⁰ represents (1) H, (2) lower alkyl which may be substituted with a group selected from the group consisting of aryl, HO-C₁₋₁₀ alkylene-O- and HO, or (3) aryl; and T¹ represents O or S), or (xi) R¹¹ -T²-(wherein R¹¹ represents (1) OH, (2) R⁷R⁸N-, (3) lower alkyl-O-, (4) lower alkyl, (5) aryl, or (6) a heterocyclic group; and T² represents CO or SO₂).

9. The compound or a pharmaceutically acceptable salt thereof according to claim 8, wherein the formula (I) is a compound wherein
each of R¹ and R², which may be the same or different, represents (i) H or (ii) lower alkyl which may be substituted with one heterocyclic group which may be substituted; and
each of R³, R⁴, R⁵ and R⁶, which may be the same or different, represents (i) H, (ii) halogen, or (iii) lower alkyl-O-.

10. A method of treating attention deficit/hyperactivity disorder, comprising administering an effective dose of a BEC1 potassium channel inhibitor or a pharmaceutically acceptable salt thereof to a patient.

11. The method according to claim 10, wherein the BEC1 potassium channel inhibitor is a compound of the following formula (I): 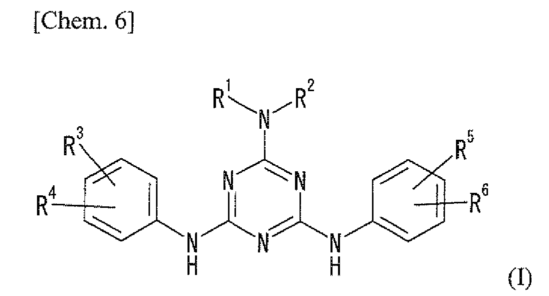 wherein the symbols are as follows;
each of R¹ and R², which may be the same or different, represents (i) H, (ii) OH, (iii) alkyl-O-, (iv) aryl-CO-, (v) H₂N, (vi) (alkyl which may be substituted with OH)-NH, (vii) (alkyl)₂N, (viii) a hydrocarbon group which may be substituted, or (ix) a heterocyclic group which may be substituted; and
each of R³, R⁴, R⁵ and R⁶, which may be the same or different, represents (i) H, (ii) CN, (iii) NO₂, (iv) halogen, (v) lower alkyl which may be substituted with a group selected from the group consisting of CN, halogen, and OH, (vi) cycloalkyl, (vii) aryl which may be substituted with lower alkyl, (viii) a heterocyclic group which may be substituted with lower alkyl, (ix) R⁷R⁸N- (wherein each of R⁷ and R⁸ may be the same or different, and represents (1) H, (2) lower alkyl which may be substituted with a group selected from the group consisting of aryl and R⁹-O-CO- (wherein R⁹ represents (1) H, or (2) lower alkyl which may be substituted with aryl)), (x) R¹⁰-T¹- (wherein R¹⁰ represents (1) H, (2) lower alkyl which may be substituted with a group selected from the group consisting of aryl, HO-C₁₋₁₀ alkylene-O- and HO, or (3) aryl; and T¹ represents O or S), or (xi) R¹¹-T²-(wherein R¹¹ represents (1) OH, (2) R⁷R⁸N-, (3) lower alkyl-O-, (4) lower alkyl, (5) aryl, or (6) a heterocyclic group; and T² represents CO or SO₂).

12. The method according to claim 11, wherein the formula (I) is a compound wherein
each of R¹ and R², which may be the same or different, represents (i) H or (ii) lower alkyl which may be substituted with one heterocyclic group which may be substituted; and
each of R³, R⁴, R⁵ and R⁶, which may be the same or different, represents (i) H, (ii) halogen, or (iii) lower alkyl-O-.
